# EUROPEAN PATENT SPECIFICATION

(11) **EP 0 778 890 B1**
(45) Date of publication and mention of the grant of the patent: **27.09.2000**
(21) Application number: 94925144.1
(22) Date of filing: 29.07.1994
(51) Int. Cl.: C12N 15/12, C07K 14/705, C12N 5/10, G01N 33/50

(54) **DNA ENCODING A HUMAN CALCIUM CHANNEL ALPHA-1E SUBUNIT**
FÜR DIE ALPHA-1E UNTEREINHEIT DES MENSCHLICHEN KALZIUMKANALS KODIERENDE DNA
ADN CODANT UNE SOUS-UNITE ALPHA-1E DE CANAL CALCIQUE HUMAIN

(30) Priority: 30.07.1993 US 100740
(43) Date of publication of application: 18.06.1997
(73) Proprietor: Elan Pharmaceuticals, Inc., South San Francisco, CA 94080 (US)
(72) Inventor: HORNE, William A., San Carlos, CA 94070 (US); BELL, John R., Redwood City, CA 94065 (US); CONG, Ruth, Oakland, CA 94606 (US); HASHIMOTO, Chika, Menlo Park, CA 94025 (US); PALMA, Andrew, San Ramon, CA 94583 (US); PHILIP, Mohan, Redwood City, CA 94061 (US); ZHOU, Mei, Menlo Park, CA 94025 (US)
(74) Representative: Vossius, Volker, Dr.
(86) International application number: US9408589
(87) International publication number: WO9504144

(56) References cited:
- EP-A- 0 507 170
- WO-A-95/04822
- 23RD ANNUAL MEETING OF THE SOCIETY FOR NEUROSCIENCE, WASHINGTON, D.C., USA, NOVEMBER 7-12, 1993. SOCIETY FOR NEUROSCIENCE ABSTRACTS, 19 (1-3). 1993. 11., XP002038497 MARUBIO L ET AL: "Cloning and functional expression of human alpha-1E high-voltage activated calcium channel"
- SCIENCE, Volume 260, issued 21 May 1993, T.W. SOONG et al., "Structure and Functional Expression of a Member of the Low Voltage-Activated Calcium Channel Family", pages 1133-1136.
- GENBANK SEQUENCE DATABASE RECORD, Accession No. L15453, issued 28 May 1993.
- NEURON, Volume 8, issued January 1992, M.E. WILLIAMS et al., "Structure and Functional Expression of alpha-1, alpha-2 and beta Subunits of a Novel Human Neuronal Calcium Channel Subtype", pages 71-84.
- SCIENCE, Volume 231, issued 07 March 1986, N. DASCAL et al., "Expression and Modulation of Voltage-Gated Calcium Channels After RNA Injection in Xenopus Oocytes", pages 1147-1150.

## Description

### Field of the Invention

The present invention relates to human calcium channel compositions. In particular, the invention includes compositions containing a human neuronal calcium channel alpha subunit designated subtype "1E" herein. Compositions of the invention include coding sequences for the subunit, as well as cells containing such coding sequences and expressing the human neuronal alpha-1E subunit.

### References

Ausubel, F.M., *et al*. (1992) Current Protocols in Molecular Biology, John Wiley and Sons, Media, PA.
Dubel, *et al*. (1992) *Proc. Natl. Acad. Sci.* 89:5058-5062.
Harpold, M.M., *et al*. (1993) PCT Patent Application Pub. No. WO 93/04083.
Hulin, R., *et al*. (1990) *EMBO J*. 11:885-890.
Mintz, I.N., *et al*. (1991) *Proc. Natl. Acad. Sci. USA* 88:6628-6631.
Mori, T, *et al*. (1991) *Nature* 350:398-402.
Sambrook, *et al*. (1989) Molecular Cloning, A Laboratory Manual, Cold Spring Harbor Laboratory Press.
Scharf, S. J., et al., Science 233:1076 (1986).
Snutch, T.P., *et al*. (1990) *Proc. Natl. Acad. Sci. USA* 87:3391-3395.
Tanabe, T. et al (1987) Nature 328:313-318.
Tsien, R.W., *et al*. (1991) *TIPS* 12:349-354.
Williams, M. E. *et al*. (1992) Neuron 8:71-84.

### Background of the Invention

Voltage-gated calcium channels are present in a wide variety of tissues, particularly excitable tissues, where they act to regulate membrane excitability. Such functions as muscle contraction and synaptic transmission are regulated, at least in part, by specific voltage-gated calcium channels. Compounds, such as dihydropyridine compounds, that block certain of these channels are used therapeutically in the management of various disorders such as hypertension, angina and subarachnoid hemorrhage.

Voltage-gated calcium channels have generally been classified according to their electrophysiological and pharmacological properties. Currently, at least four classes of voltage-gated calcium channels are recognized: L-type, T-type, N-type, and P-type. However, molecular cloning techniques have revealed the existence of different sub-types of channel within some of these classes. (see Tsien et al., supra for review).

In general, voltage gated calcium channels have been shown to consist of at least 4 non-identical subunits: the alpha-1 subunit, alpha-2 subunit, beta subunit and gamma subunit. For the L-type calcium channels, which are probably the best characterized calcium channels, it has been shown that the alpha-1 subunit contains the binding site for dihydropyridine ligands.

Partial cDNA clones encoding portions of several different subtypes of of the rat neuronal calcium channel alpha-1 subunit, referred to as rat brain class A, B, C and D, were first isolated from rat brain cDNA libraries (Snutch *et al*., supra).

The rat brain calcium channel α₁ subunit E-II was described by Soong et al., 1993, Science 260: 1133-1136. Homologous alpha-1 clones subtypes A-D have been described in humans by Harpold et al. (PCT/US92/06903; WO93/04083).

The present invention is concerned with a fifth human neuronal calcium channel alpha-1 subunit, termed "1E" or hα-1E herein. This subunit diverges considerably from the other human alpha-1 subunit clones, exhibiting only about 62% deduced amino acid sequence homology to other human neuronal alpha-1 subunits. As shown herein, this subunit can form a heterologous calcium channel with alpha-2 and beta subunits. The novel channel has a unique pharmacology and is therefore useful in screening for new, more selective therapeutic agents directed at calcium channel modulation.

### Summary of the Invention

In one aspect, the invention includes an isolated DNA fragment that encodes an alpha-1E subunit of a human neuronal calcium channel. The fragment has the nucleotide sequence presented as SEQ ID NO:1.

Also included in the invention is an alpha-1E polypeptide subunit of a human neuronal calcium channel having the sequence presented as SEQ ID NO:2. In another aspect, the invention includes a mammalian expression vector containing the nucleotide sequence SEQ ID NO:1.

A eukaryotic cell in acordance with the invention includes a heterologous DNA having the sequence presented as SEQ ID NO:1. The cell expresses a neuronal calcium channel including a polypeptide subunit having the sequence SEQ ID NO: 2. The cells may be further designed to express neuronal calcium channel subunits α₂ and β.

The cell line can be used to screen compounds effective to inhibit calcium uptake by neuronal cells.

These and other objects and features of the invention will be more fully appreciated when the following detailed description of the invention is read in conjunction with the accompanying drawings.

### Brief Description of the Drawings

Figure 1 shows the DNA sequence (SEQ ID NO: 1) of the human neuronal calcium channel alpha subunit hα-1E and the deduced amino acid sequence (SEQ ID NO: 2) for the channel subunit; and
Figure 2 shows a tracing of a barium current measured in a eukaryotic cell expressing a heterologous calcium channel including the human neuronal calcium channel alpha subunit hα-1E.

### Detailed Description of the Invention

### I. Definitions

Unless otherwise indicated, all terms used herein have the same meaning as they would to one skilled in the art of the present invention. Practitioners are particularly directed to Current Protocols in Molecular Biology (Ausubel, et al., supra) for definitions and terms of the art.

The terms "heterologous DNA" and "heterologous RNA" refer to nucleotides that are not endogenous to the cell or part of the genome in which they are present; generally such nucleotides have been added to the cell, by transfection, microinjection, electroporation, or the like. Such nucleotides generally include at least one coding sequence, but this coding sequence need not be expressed.

The term "expression vector" refers to vectors that have the ability to incorporate and express heterologous DNA fragments in a foreign cell. Many prokaryotic and eukaryotic expression vectors are commercially available. Selection of appropriate expression vectors is within the knowledge of those having skill in the art.

### II. Isolation of DNA Coding Sequences for Human Neuronal Alpha-1E Subunit

The diversity of voltage-gated calcium channels between and within tissues as well as across species is becoming more apparent, as new channels having distinct electrophysiological and pharmacological characteristics are reported. The present invention describes a new calcium channel isolated from human brain. As reported herein, this channel is categorized as an alpha subunit of a voltage-gated calcium channel. The novel human channel reported herein is referred to as the human alpha-1E subunit, or hα-1E, in accordance with the nomenclature defined by Snutch et al., supra for rat brain-derived calcium channel alpha subunits A-D.

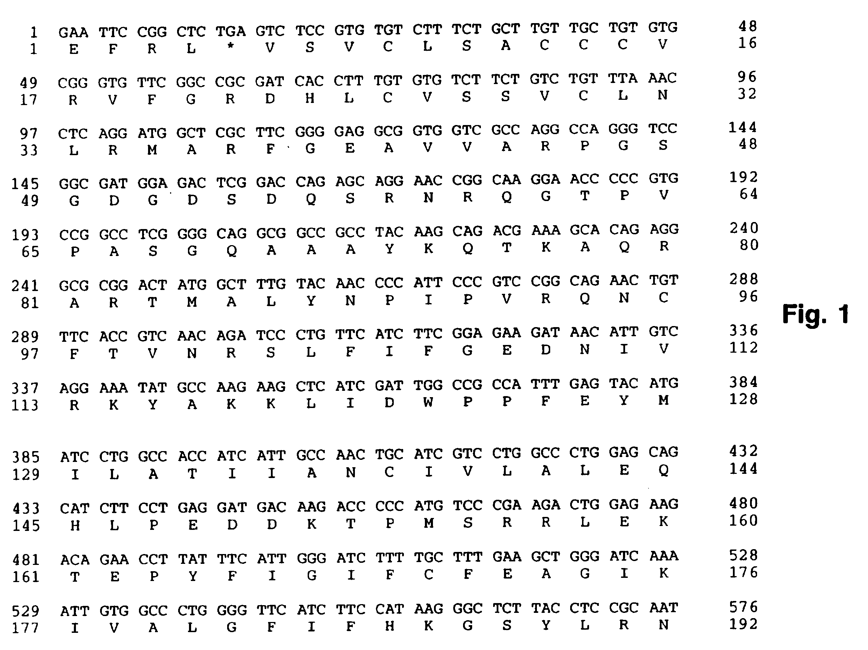

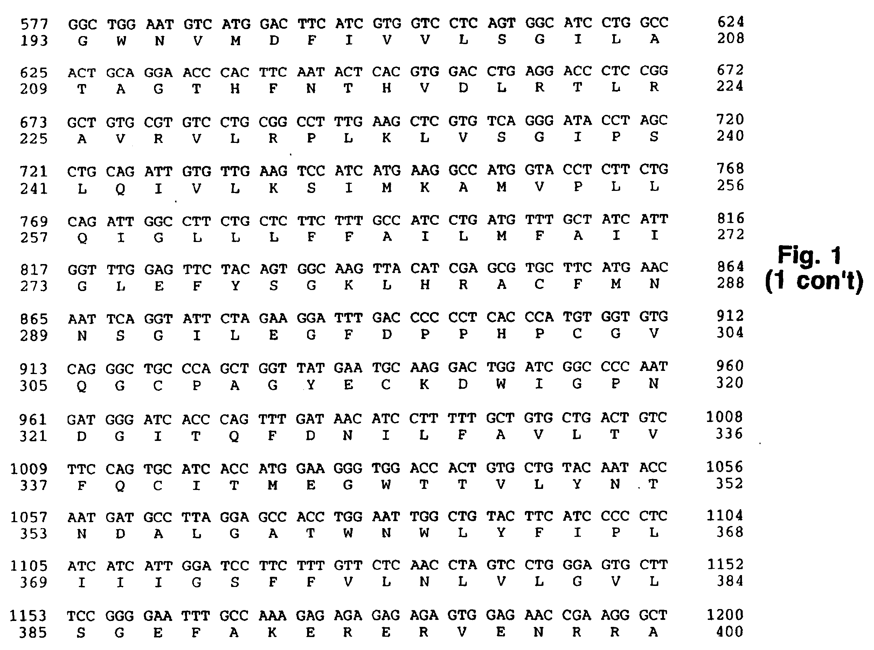

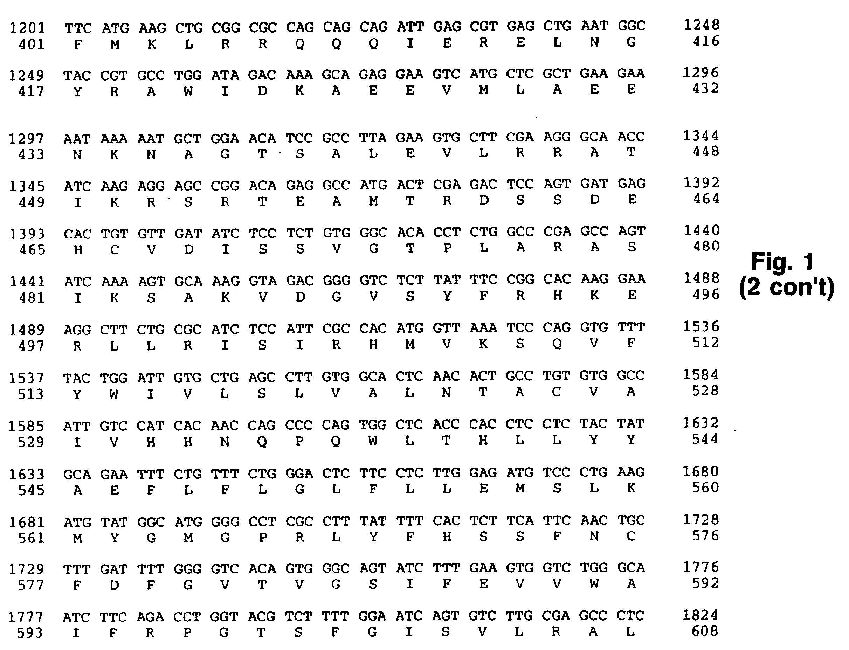

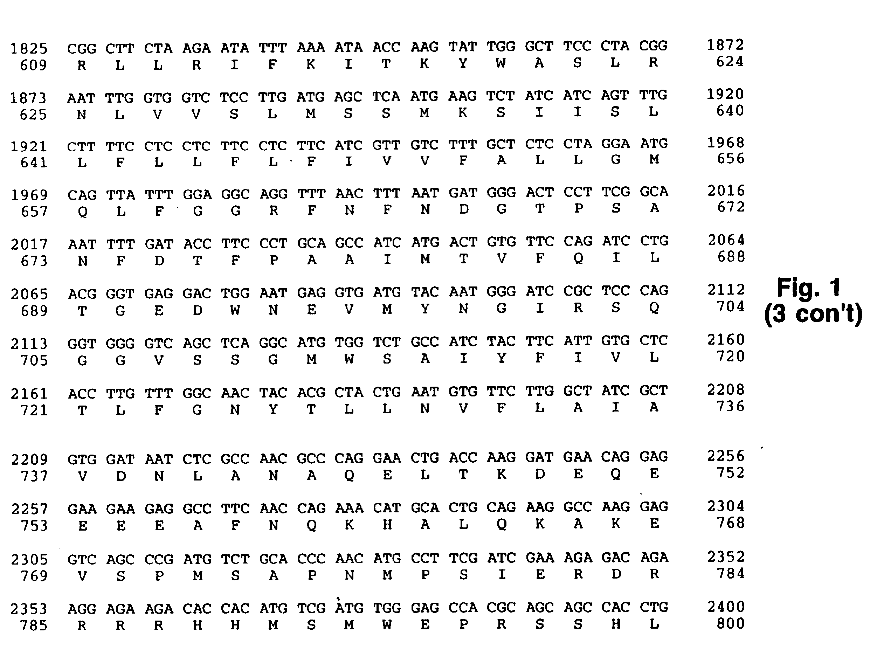

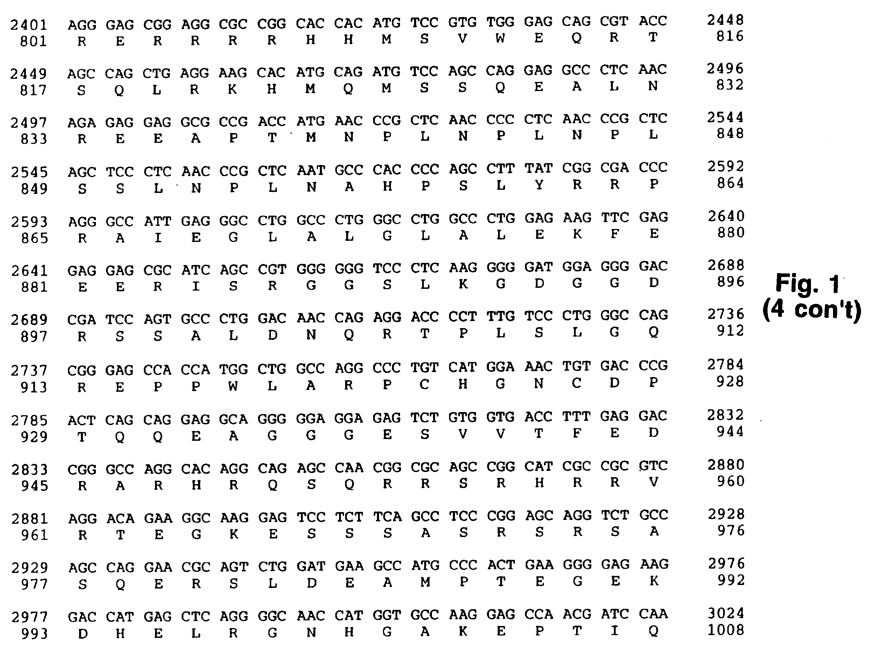

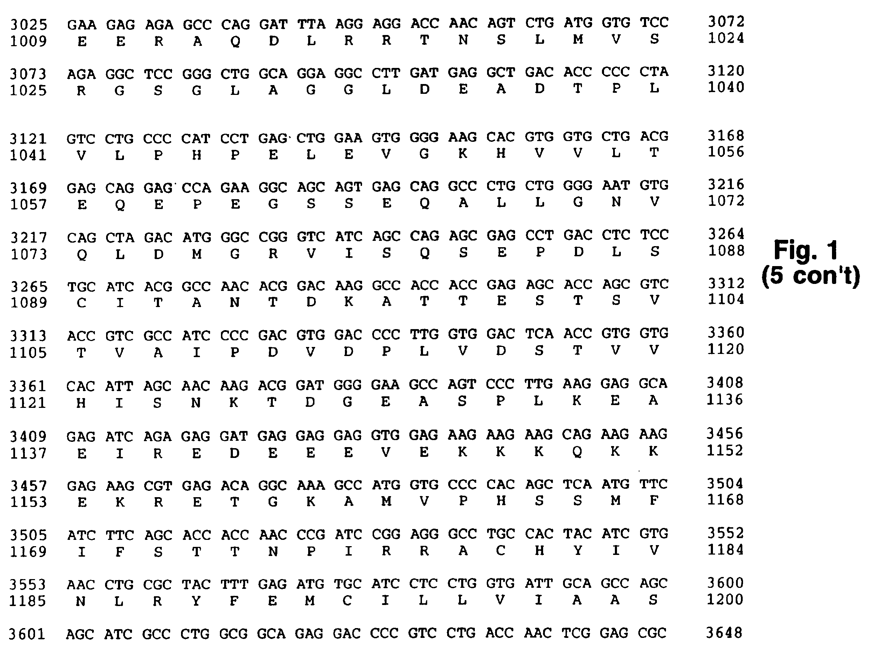

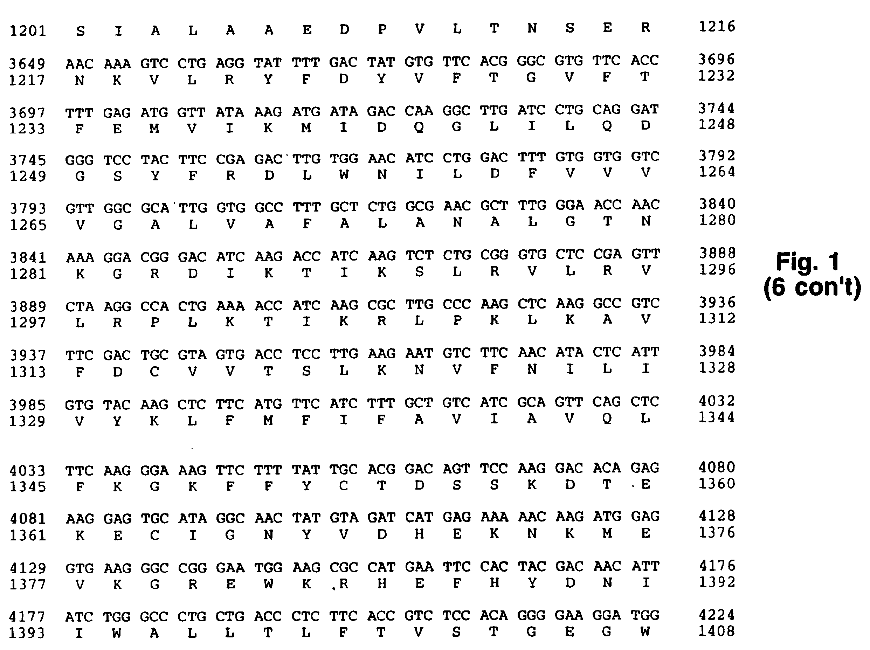

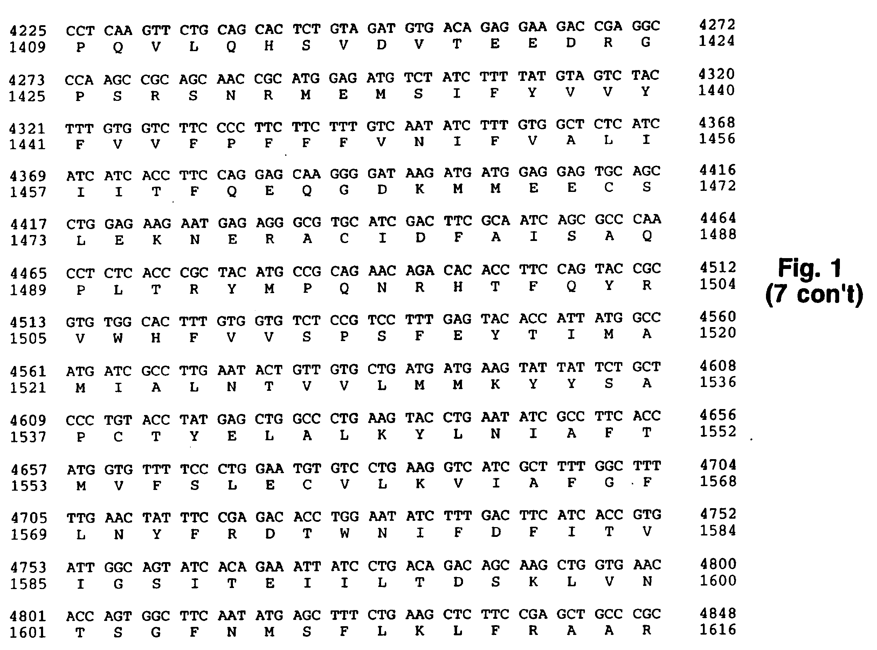

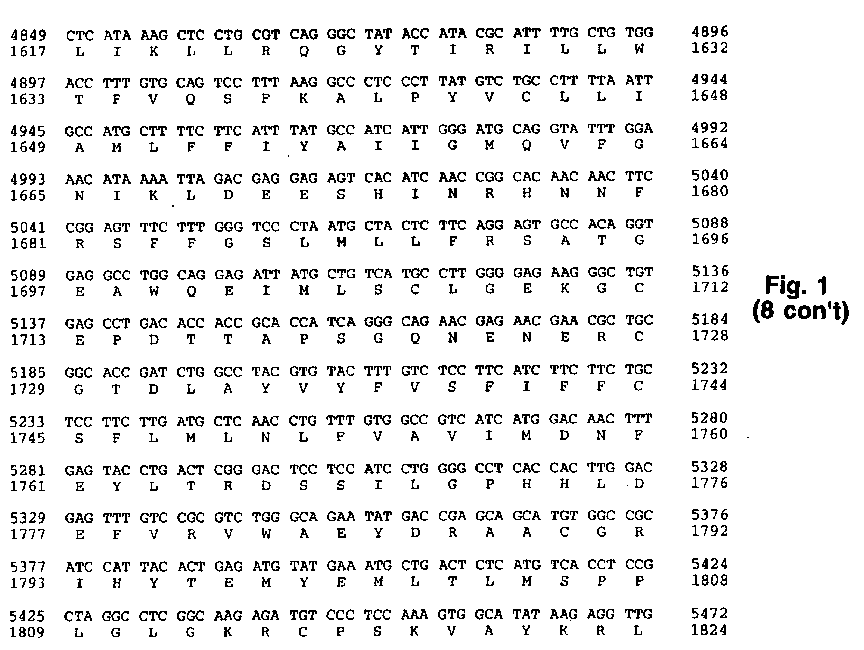

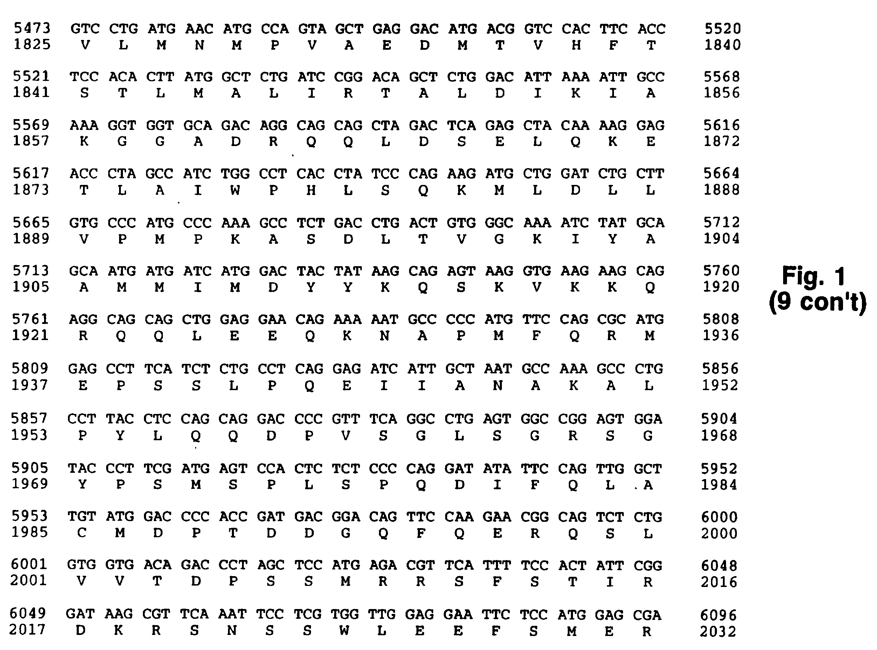

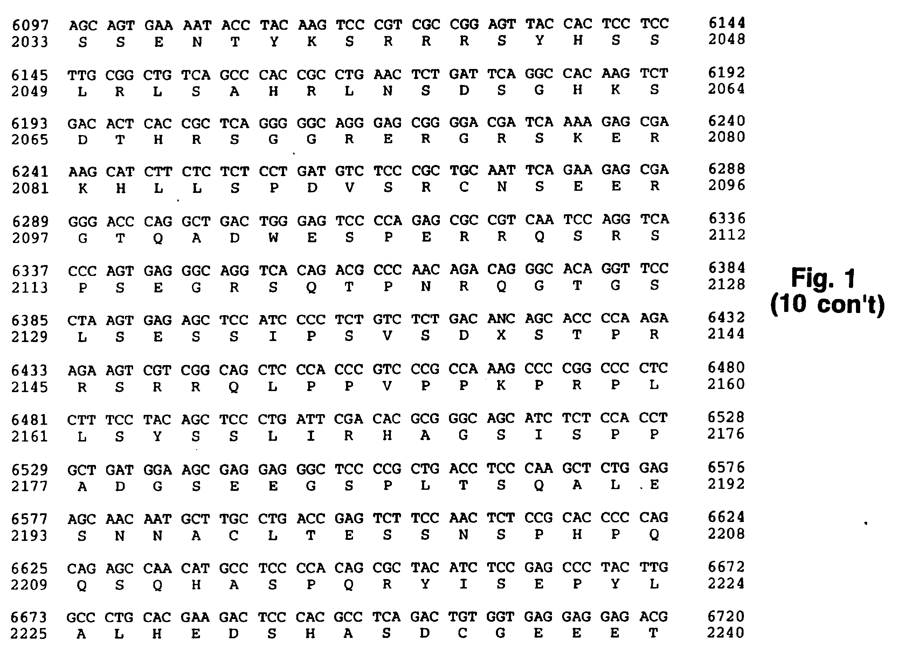

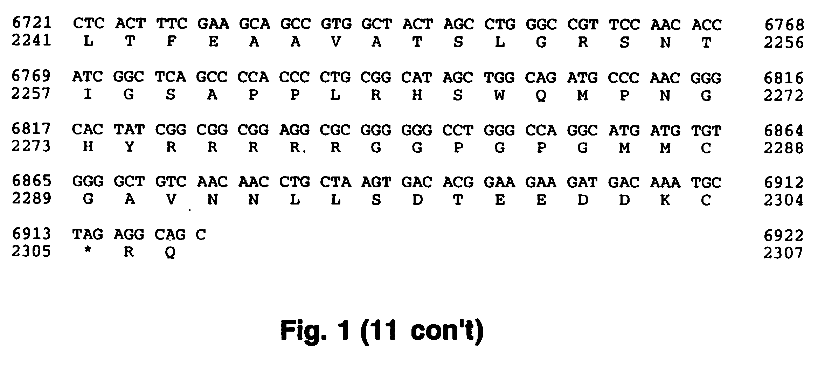

A number of overlapping partial cDNA clones were isolated from a human hippocampal library in order to characterize the complete hα-1E coding sequence. The complete hα-1E nucleotide (nt) sequence is depicted in Figure 1 as SEQ ID NO: 1. Also shown is the deduced amino acid sequence (single letter code indicated below nt sequence) as SEQ ID NO: 2. A list of the of four partial cDNA clones used to characterize the α1E sequence and the nucleotide position of each clone relative to the full-length hα-1E sequence (SEQ ID No.1) is shown in Table 1 below.

**Table 1**

| Identification of Partial hα-1E Clones | | |
|---|---|---|
| Name | SEQ ID NO | Location Relative to Full-Length Sequence |
| H24 | 3 | nt 1 to 2374 of SEQ ID No 1 |
| H6 | 4 | nt 1185 to 5377 of SEQ ID No 1 |
| I2 | 5 | nt 3192 to 6435 of SEQ ID No 1 |
| 3-69A | 6 | nt 5176 to 6922 of SEQ ID No 1 |

The isolation and characterization of each of the partial clones are described below and detailed in Example 1. The codon for the start methionine begins with nt 107 and nt 6912 represents the end of the open reading frame. The initial 107 and final 10 nucleotides (nt) represent 5' and 3' untranslated regions, respectively.

### A. Screening Libraries for Human Calcium Channels

Synthetic oligonucleotide probes for hybridization screening were prepared on an automated oligonucleotide synthesizer (Applied Biosystems, Foster City, CA). In order to obtain clones to form the complete hα-1E sequence, oligonucleotide probes based on other calcium channels were constructed as described in Example 1. Probes were constructed based on the sequence of the rat calcium channels such as those described by Snutch et al. and Dubel et al., supra. These subunits are alpha-1 subunits of rat neuronal calcium channels. They exhibit only about a 62% amino acid sequence identity with the hα-1E clone which was eventually constructed. Further probes were restriction fragments of partial clone H6.

A human hippocampal library was obtained from a commercial supplier (Stratagene, La Jolla, CA). Alternatively, brain libraries from the hippocampal or other discrete brain regions, or from the whole brain, can be produced according to standard methods now known in the art (Ausubel et al., supra), in order to identify variants, such as splice variants of the hα-1E channel. Such methods include lysing of cells comprising the region of interest to extract RNA. PolyA+ RNA is Then isolated and used to synthesize single-stranded cDNA, from which is produced double-stranded cDNA, using specific or random hexamer primers, such as hexadeoxynucleotide primers (Clontech, Palo Alto, CA).

Restriction enzyme adaptor regions are then ligated to the single-stranded cDNA, according to standard methods (Sambrook et al,. supra). Such cDNA is then purified and size-selected by agarose gel chromatography. The cDNA is then eluted and ligated to a selected vector sequence, such as a lambda gt11 vector or a lambda ZAP vector, as used in the Examples reported herein. The vector is then packaged into appropriate phage hosts and used to infect bacterial cells, as E.coli according to methods known in the art.

Commercial or custom generated libraries, such as phage-cDNA libraries described above, are screened using as probes oligonucleotide hybridization probes, as described in Example 1, under standard conditions, and medium stringency (Ausubel et al., supra). Briefly, oligonucleotide hybridization probes are radiolabeled by random priming methods (Sambrook et al., supra), then hybridized to immobilized DNA from the cDNA library. Those phage plaques showing hybridization with the probes are selected, subcloned, and re-tested. Positive clones are identified by autoradiography. Clones identified according to the methods described above are expected to be partial sequence clones, due to the size selection of the cDNA used in generating the library and the predicted size of the calcium channel alpha subunit, based on data from calcium channels from other species.

Clones identified and isolated as described above are placque purified prior to extraction of DNA and production of double stranded plasmid cDNA. The sequence of the plasmid cDNA is then determined, by standard methods, such as on a commercial DNA sequencer (Applied Biosystems, Foster City, CA). As noted above, four partial clones, H6, H24, I2, and 3-69A, were used to determine the full-length sequence of hα-1E. Regions of overlap between different clones obtained were determined by comparison of the sequences.

Alternatively, the sequences provided by the end-terminal sequences of partial clones useful as specific sequence primers in first-strand DNA synthesis reactions (Maniatis et al., supra; Scharf et al., supra) using, for example, partially purified total cellular RNA as substrate. Synthesis of the second-strand of the cDNA is randomly primed (Boehringer Mannheim, Indianapolis IN). The above procedures identify or produce cDNA molecules corresponding to nucleic acid regions that are adjacent to any of the partial clone sequences. These newly isolated sequences can in turn be used to identify further flanking sequences, and so on, to identify overlapping cDNA clones from which the entire hα-1E sequence can be determined. Using the general methods described above, and detailed in Example 1, the sequence of the full-length hα-1E coding sequence is determined. The full-length hα-1E clone is constructed from the partial overlapping clones is detailed in Example 2.

### B. Construction of Full-length hα-1E Clones

With reference to Table 1 and Figure 1, it can be seen that α1E cDNA clones H24, H6, I2, and 3-69A, isolated as described above, overlap and include the nucleotide sequence which codes for the entire α1E open reading frame, nt 107 to 6912 (SEQ ID No: 1). Restriction fragments of these partial cDNA clones were ligated together to generate a full-length α1E cDNA in a eukaryotic expression vector (pcDNA III- Invitrogen). The resulting construct was named NX-HE1.

The construction of hα-1E, termed NX-HE1 and identified as SEQ ID NO: 1, herein, was performed in multiple steps as described in detail in Example 2. Briefly, the H24 clone (SEQ ID NO:3) was recloned into Bluescript SK+ ("BS") (Stratagene, San Diego, CA) to obtain proper orientation within the BS polylinker such that the BS XhoI site was 5' relative to the coding region, forming Construct 1. An XhoI fragment from Construct 1 was then ligated into the eukaryotic expression vector pcDNA III (Invitrogen, San Diego, CA) to form Construct 2. An XhoI/ApaI fragment from clone H6 (SEQ ID NO: 4) was then ligated into Construct 2 form Construct 3. The remainder of the 3' end was constructed separately, as follows. An SfiI/BamHI fragment from clone 3-69A was ligated into clone I2 (SEQ ID NO: 5) to form Construct 4. Finally, an ApaI fragment of Construct 4 was ligated into Construct 3 to from the full length clone SEQ ID No.1). This ligated product containing the full length sequence was named NX-HE1.

### III. Heterologous Expression of hα-1E in Cells

It can be appreciated that, given the diversity and importance of voltage-gated calcium channels in mammalian physiology, possession of cells which transiently or constitutively express selected channel subtypes, such as the hα-1E subtype calcium channel, would find use in the medical arts, particularly in the areas of diagnosis and/or drug screening. Exemplary assays in these areas are described in Section V.

It can be appreciated that functional expression of calcium currents is particularly useful in practicing parts of the invention described herein. However, expression of a particular heterologous DNA sequence can be monitored to some advantage, using non-functional assays, such as Northern blot assays and protein expression assays, such as immunodetection methods. The present invention provides tools for use in such methods, including oligonucleotide probes and proteins and peptides for production of antibodies for use in immunodetection assays.

### A. Preparation of Recombinant Eukaryotic Cells Containing DNA Encoding Heterologous hα-1E Subunits

DNA encoding the hα-1E calcium channel subunit may be introduced into a host cell for expression of the DNA. Methods for introduction of such DNA into cells are known those skilled in the art (Ausubel et al., supra), Sambrook et al., supra). Such methods include, for example, transfection of eukaryotic cells with an appropriate expression plasmid vector, such as the vectors described in Section II herein, or a combination of plasmid vectors each containing a different calcium channel subunit, selected from alpha-1, alpha-2, beta, and gamma subunits known in the art to form functional calcium channels (Williams et al., supra) each encoding one or more distinct genes or with linear DNA, and selection of transfected cells are also well-known in the art (Sambrook *et al*., supra).

Cloned full-length DNA encoding the alpha-1E subunit of a human calcium channel, such as the hα-1E DNA sequence SEQ ID NO: 1, described herein, may be introduced into a plasmid vector for expression in a eukaryotic cell. Host cells may also be transfected with linear DNA according to standard methods.

Practice of the present invention can be effectively carried out using any of a number of mammalian expression systems, including yeast cells. However, mammalian expression systems may be preferred for practicing certain aspects of the invention.

Eukaryotic cells suitable for introduction of heterologous hα-1E calcium channel subunit include any cells that are transfectable by such DNA or RNA or into which such DNA may be injected. Preferred host cells are those that can also express the heterologous DNA and RNA. For practicing certain aspects of the invention, such as electrophysiological measurements described in Section IV it is appreciated that it may be desirable that the host cell lack endogenous functionally expressed voltage gated calcium channels having current characteristics similar to those exhibited by the human alpha-1 calcium channel subunits described herein. In such cases it may be necessary, in order to observe and measure functional expression of the exogenously added alpha-1E subunit, to introduce into the cell heterologous coding sequences encoding the alpha-2 and possibly also the beta calcium channel subunit. As described above, coding sequences for such auxilliary subunits have been published, and expression in vectors suitable for introduction into cells is well within the skill of the practitioner.

In addition, preferred cells for introducing DNA include those that can be transiently or stably transfected and include, but are not limited to, cells of mammalian origin, such as COS cells, mouse 1 cells, Chinese Hamster Ovary (CHO) cells, human embryonic kidney (HEK) cells, African green monkey cells, and the like. Preferred cells include DG44 cells and HEK 293 cells, particularly HEK 293 cells that have been adapted for growth in suspension. Additionally *Xenopus Laevis* oocytes find use in the practice of the invention, as described in Part B and Section IV, below. Additionally, yeast cells such as *Saccharomyces cerevisiae* or *Pichia pastoris* may be utilized in practicing the invention.

Heterologous DNA encoding a human alpha-1E subunit, such as the hα-1E calcium channel subunit, may be introduced by any method known to those skilled in the art, such as transfection with a vector containing the DNA sequence that encodes the subunit, such as the DNA sequence SEQ ID NO: 1. Particularly preferred vectors for transfection of mammalian cells are the pSV2dhfr expression vectors, which contain the SV40 early promoter, mouse dhfr gene, SV40 polyadenylation and splice sites and sequences necessary for maintaining the vector in bacteria, cytomegalovirus (CMV) promoter-based vectors such as pCMV or pCDNA1 or PcDNA3 (Invitrogen), and MMTV promoter-based vectors. DNA encoding the human calcium channel subunit hα-1E is been inserted in the vector pcDNA3 such that its expression is regulated by the CMV promoter. Such constructs are suitable for transfecting a number of mammalian cells, including COS cells and HEK 293 cells. Other suitable vectors and cell targets include, but are not limited to pCMV and pREP vectors (obtained from Invitrogen, San Diego, CA).

Stably or transiently transfected mammalian cells may be prepared by methods that are well known in the art. Cells are transfected with an expression vector having a selectable marker gene such as the gene for thymidine kinase, dihydrofolate reductase, or the like. Stable transfection of cells is conveniently achieved by growing the transfected cells under conditions that promote growth of cells expressing the marker gene.

The heterologous DNA encoding the human alpha-1E calcium channel subunit may be integrated into cell's chromosomal material or may be maintained in the cell as an episomal element. Cells containing such heterologous DNA may be passaged and/or subcultured, according to methods appropriate to the cell type and known in the art.

### B. Functional Expression of hα-1E in Xenopus oocytes

A plasmid, such as a pBluescript SK+ plasmid, containing the full-length NX-HE1 coding sequence is used to produce complementary RNA, using an appropriate RNA polymerase, such as T7 or SP6 polymerase. Such RNA is injected into oocytes from *Xenopus laevis* (about 5-10 pg/oocyte), according to methods known in the art. For expression of a funtional calcium channel in oocytes, coexpression of a calcium channel alpha-2 subunit, such as the rabbit skeletal muscle alpha-2 subunit (Mori et al., supra) may be required. Additionally, inclusion of a calcium channel Beta subunit, such as the Beta-3 subunit from rabbit heart (Hulin et al, supra), helps optimize functional expression of the channel. Coding sequences for the aforementioned alpha-2 and beta-3 subunits have been published as cited above, and construction of expression vectors suitable for injection into oocytes is within the skill of the practitioner. For expression in oocytes, approximately equimolar amounts of RNA are injected into each oocyte.

### C. Functional Expression of hα-1E in Mammalian Cells

One cell type that is particularly amenable to transient or stable transfection is the human embryonic kidney cell line HEK 293 (ATCC Accession No. CRL1573). Such cells can be transiently cotransfected with the hα-1E subunit cDNA expression plasmid, and one or more of an α₂ calcium channel subunit cDNA expression plasmid, an β₁ subunit cDNA expression plasmid as detailed in Example 4 (Williams et al., supra).

Stable transfection can also be achieved in HEK 293 cells according to standard methods (Ausubel et al., supra). Suitable vectors for stable transfection include pcDNA1 and pcDNA3.

Transfected cells are selected, for example by differential growth in limiting medium, according to methods known in the art. Such cells are subcloned and tested, for example by Northern blot analysis, for the evidence for the expression of the human alpha-1E calcium channel. Alternatively or in addition, individual transfected cells can be analyzed electrophysiologically for the presence of voltage-activated calcium currents (using barium as carrier, as described in Section IV. Such cells are useful in functional and/or binding assays, as described in Section V.

### IV. Electrophysiological Measurements

### A. Recording of Calcium currents in Xenopus oocytes

Functional expression of a heterologous hα-1E calcium channel subunit can be measured in *Xenopus* oocytes injected with heterologous RNA, as conveniently recorded using a standard two-microelectrode voltage-clamp connected to an amplification system. Cells are placed in a chloride-free bathing solution containing about 40 mM Ba(0H)₂, such that barium acts as current carrier in the system. Channels are activated by periodic delivery of voltage pulses. Currents are measured and normalized according to procedures known to those familiar with the art pertaining to electrophysiology.

### V. Utility

The present invention includes methods for identifying therapeutic compounds, such as calcium channel agonist and antagonists, that modulate the activity of calcium channels. Such assays are useful as screens for new therapeutic compounds having calcium channel agonist and/or antagonist activity. Presently, calcium channel antagonists directed at L-type calcium channels find use in the treatment of hypertension, subarachnoid hemorrhage and variant forms of angina. Certain N-type calcium channel antagonists have been found to be useful in reducing cerebral ischemia, as described in co-owned U.S. Patents 4,051,403 and 5,189,020.

According to the present invention, eukaryotic cells expressing heterologous hα-1E calcium channel subunits encoded by heterologous DNA as described herein are useful for screening for hα-1E subtype-specific compounds, and for predicting the relative potencies of such compounds.

In particular, cells expressing such heterologous calcium channels of the hα-1E subtype can be used in binding assays, wherein whole cells or membranes thereof are tested for ability to bind a test compound, generally by measuring the ability of the test compound to displace a known ligand of the channel. One appropriate functional ligand which binds to the channel and blocks hα-1E calcium currents is the spider toxin Aga-IIIA (isolated as described by Mintz et al., supra).

Methods for carrying out such screening assays are well known in the art, and setting up such assays is within the skill of the practioner. Co-owned allowed U.S. patent application 07/855,269, describes assays which utilize isolated neuronal calcium channels for screening of certain types of N-type calcium channel antagonists. Such methods are adaptable to the present invention.

Alternatively, or additionally, such screening assays may include assays in which is determined the functional activity of an expressed calcium channel. In such an assay, drugs which alter such activity, such as calcium current activity, are candidates for calcium channel-based therapeutics, of the types suggested above or or additional types.

The following examples illustrate, but in no way are intended to limit the present invention.

### Materials and Methods

A lambda-ZAP cDNA human hippocampal cDNA library was obteined from Stratagene (La Jolla, CA). A eukaryotic expression vector pcDNA III was obtained from Invitrogen (San Diego CA). T4 DNA ligase and T4 DNA polymerase were obtained from New England Biolabs (Beverly, MA); Nitrocellulose filters were obtained from Schleicher and Schuell (Keene, NH).

Bluescript SK+(BS) was obtained from Stratagene (San Diego, CA).

Dephosphorylated Calf intestinal phosphatase (CIP) was obtained form New England Biolabs (Beverly, MA).

Synthetic oligonucleotide linkers and primers were prepared using commercially available automated oligonucleotide synthesizers, such as obtained from Applied Biosystems (Foster City, CA). Alternatively, custom designed synthetic oligonucleotides may be purchased, for example, from Synthetic Genetics (San Diego, CA). cDNA synthesis kit and random priming labeling kits were obtained from Boehringer-Mannheim Biochemical (BMB, Indianapolis, IN).

Standard molecular biology and cloning techniques were performed essentially as previously described in Ausubel *et al*., Sambrook *et al*., and Maniatis *et al*., supra.

### Example 1

### Isolation of partial cDNA clones

### A. Clone H6

One million recombinants of a λZAP II (Stratagene, La Jolla, CA) human hippocampal cDNA library were screened in duplicate at a density of 50,000 plagues per 150 mm plate using two radiolabeled 1.6 kb (Hind III and Xho I digested) fragments of the rat Class B α1 subunit cDNA (for the sequence of the rat Class B α1 subunit see Dubel *et al*., supra):

| Fragment | Nucleotides |
|---|---|
| HindIII-HindIII | 712 to 2288 |
| Xhol-Xhol | 3793-5394 |

The hybridization was performed under standard conditions (5x SSPE, 5X Denhardt's, 0.1% SDS, 1 mg/ml salmon sperm DNA; recipes found in Sambrook *et al*., supra). Filters were washed under medium stringency conditions (0.2X SSPE, 0.1% SDS, 50°C). H6 was one of two Class E specific clones isolated. H6 bacteriophage was plaque purified using standard methods (Sambrook *et al*., supra), double stranded BS plasmid CDNA was isolated using standard phagemid rescue procedures (Stratagene), and DNA sequence was obtained using PCR based fluorescence dye termintaor procedures (Applied Biosystems).

### B. Clone H24

One million recombinants of a λZAP II (Stratagene) human hippocampal cDNA library were screened in duplicate at a density of 50,000 plaques per 150 mm plate using a radiolabeled NotI/EcoRI fragment (nucleotides 118 to 705) of the rat Class B α1 subunit cDNA (for the sequence of the rat Class B α1 subunit see Dubel *et al*., supra). The hybridization was performed under standard conditions. Filters were washed under medium stringency conditions H24 was one of 5 positives isolated in the screen (4 of which were Class E clones). H24 bacteriophage was plaque purified, double standard plasmid cDNA was prepared, and the clone was chartacterized by DNA sequencing.

### C. Clone I2

One million recombinants of a λZAP II (Stratagene) human hippocampal cDNA library were screened in duplicate at a density of 50,000 plaques per 150 mm plate using a radiolabeled PstI/ ApaI fragment (nucleotides 4194 to 4740) from the CDNA fragment H6. The hybridization was performed under standard conditions. Filters were washed under medium stringency conditions. I2 was one of 13 positives isolated in the screen. I2 bacteriophage was plaque purified, double standard plasmid cDNA was prepared, and the clone was chartacterized by DNA sequencing.

### D. Clone 3-69A

One million recombinants of a λZAP II (Stratagene) human hippocampal cDNA library were screened in duplicate at a density of 50,000 plaques per 150 mm plate using a radiolabeled DNA oligonucleotide probe derived from a rat neuronal alpha subunit cDNA. The hybridization was performed under standard conditions. Filters were washed under medium stringency conditions. 3-69A was one of 37 positives isolated in the screen. 3-69A bacteriophage was plaque purified, double standard plasmid cDNA was prepared, and the clone was chartacterized by DNA sequencing.

### Example 2

### Construction of full length Human α1E cDNA

hα-1E cDNA clones H24, H6, I2, and 3-69A overlap and include the nucleotide sequence which codes for the entire hα-1E open reading frame, nt 107 to 6912 (SEQ ID No: 1). Restriction fragments of these partial cDNA clones were ligated together to generate a full-length hα-1E cDNA in a eukaryotic expression vector (pcDNA III-Invitrogen). The resulting construct was named NX-HE1. The construction of NX-HE1 was performed in multiple steps as described in detail below: 1) recloning of H24 into Bluescript SK+(BS) to obtain proper orientation (Construct 1), 2) ligation of an XhoI fragment (nt 1 to 1374) from Construct 1 into pcDNA III (to form Construct 2), 3) ligation of an XhoI/ApaI fragment from H6 (nt 1374 to 4181) into Construct 2 (to form construct 3), 4) ligation of an SfiI/BamHI fragment from 3-69A (nt 5884 to 3' untranslated) into I2 (to form Construct 4), and 5) ligation of an ApaI fragment (nt 4181 to 3' untranslated into Construct 3 to from the full length clone SEQ ID No.1).
1) To obtain Construct 1, H24 was digested with EcoRI and religated into EcoRI digested BS. The purpose of this ligation was to reorient the original H24 clone within the BS polylinker such that the BS XhoI site was 5' relative to the coding region.
2) construct 2 was digested with XhoI and the resultant 1.4 kb fragment was purified from an agarose gel and ligated into XhoI digested pcDNAIII dephosphorylated with calf intestinal phosphatase (CIP). This construct thus contained the 5' end of the clone (nt 1 to 1374).
3) H6 was then digested with XhoI and Apa I and the resultant 2.8 kb fragment (nt 1374 to 4181) was ligated into XhoI /ApaI digested and CIP treated Construct 2. The ligated product was referred to as Construct 3.
4) The remainder of the 3' end was constructed separately. First, 3-69A was digested with SfiI and BamHI to yield a 2.7 kb fragment (nt 5884 to 3' untranslated), which was gel purified and ligated into SfiI/BamHI digested and CIP treated I2. The ligated product was referred to as Construct 4. (All ligation junctions were sequenced prior to proceeding with the next step).
5) The final construct was prepared by digesting Construct 4 with ApaI, yielding a 4.1 kb fragment (nt 4182 to 3'ut ) which was gel purified and ligated into ApaI cut CIP treated Construct 3. This ligated product containing the full length sequence was named NX-HE1.

### Example 3

### Functional Expression of hα-1E in Xenopus oocytes

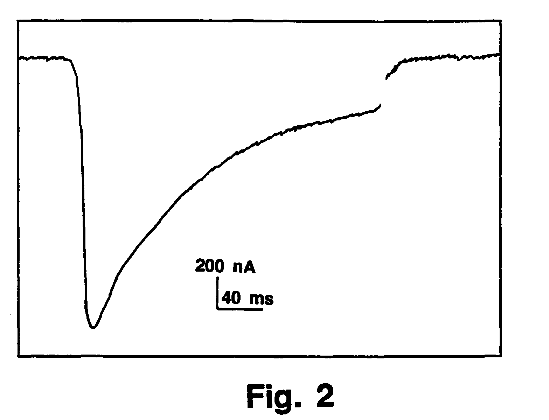

The hα-1E full-length DNA sequence SEQ ID NO:1 was subcloned into pBluescript SK + to obtain plasmid phα-1E(SK+). Complementary RNA was synthesized from the plasmid *in vitro* using T7 or SP6 polymerase. Additionally, complementary RNA is synthesized from plasmids containing calcium channel alpha-2 subunits and Beta subunits known in the art (Mori et al., Williams et al., Tanabe et al., supra). RNA from each subunit was injected in equimolar ratios using 0.1 µg ml⁻¹ α₁, 0.1 µg ml⁻¹ α₂, and 0.03 µg ml⁻¹ β; ∼ 50 nl is injected per cell. The hα-1E currents were recorded by a standard two-microelectrode voltage-clamp using a Warner amplifier (OC-725A) in a chloride free bath solution, containing (in mM): Ba(OH)₂, 5; tetraethylammonium-OH, 40; KOH, 2; HEPES, 5; pH 7.4 adjusted with methanesulphonic acid. Data were sampled at 10 kHz and filtered at 2 kHz. Leak and capacitance currents are subtracted off-line by a P/4 protocol. Voltage pulses are delivered every 15 s. For steady-state inactivation experiments, voltage pulses were delivered every 20 s. Peak normalized currents were fitted to a Hodgkin-Huxley inactivation curve. Figure 2 shows a trace of a barium current measured in a Xenopus oocyte previously injected with mRNA encoding the human calcium channel subunit hα-1E (SEQ ID NO:2) plus RNA encoding alpha-2 and beta subunits, as described above. The current was invoked from a holding potential of -80 mV to a test potential of OmV.

The spider toxin AgaIIIA was effective to block this current, at a batch concentration of 50 nM. Other calcium channel blocking compounds which were unable to block this current and maximum concentrations tested are as follows: omega conotoxins MVIIA (5 µM), MVIIC (5 µM), TVIA (5 µM), Aga IVA (100 nM) and dehydropyridines.

### Example 4

### Expression of hα-1E in Mammalian Cells

Human embryonic kidney cells (HEK 293 cells) were transiently and stably transfected with human neuronal DNA encoding calcium channel subunits.

### A. Transfection of HEK 293 Cells

Separate expression vectors containing DNA encoding human neuronal calcium channel hα-1E, rat α₂, and β₁ subunits, were used. constructed as described in Example 2. The hα-1E coding sequence described herein as SEQ ID NO: 1 was incorporated into a pcDNA1+3 vector, as described above, with addition of a c-myc epitope tag on the 5' end of the hα-1E coding sequence, for monitoring, according to established methods. The vector was used for stable transfaction of HEK 293 cells using the calcium phosphate transfaction procedure (Ausubel et al., supra). Culture plates containing about two million HEK 293 cells, were transfected with 1 ml of DNA/calcium phosphate precipitate containing 5 µg of each of the vectors. After 10-20 days of growth in media containing 500 µg G418, colonies had formed and were isolated according to standard procedures.

Expression of hα-1E subunit by cells was monitored by fluorescence immunolabeling. Cells were incubated with fluorscently tagged antibodies (from commercial sources) directed to the c-myc epitope, the coding sequence for which was added to the 5' end of the hα-1E coding sequence. Expression of the hα-1E subunit having the epitope was evidenced by immunofluorescence.

While the invention has been described with reference to specific methods and embodiments, it will be appreciated that various modifications and changes may be made without departing from the invention.

## Claims

1. An isolated DNA fragment, comprising a sequence of nucleotides that encodes an alpha-1E subunit of a human neuronal calcium channel, and having the sequence presented as SEQ ID NO:1.

2. An alpha-1E polypeptide subunit of a human neuronal calcium channel having the sequence presented as SEQ ID NO:2.

3. A mammalian expression vector containing the nucleotide sequence presented as SEQ ID NO:1.

4. A eukaryotic cell, which includes a heterologous DNA having the sequence presented as SEQ ID NO:1.

5. The cell of claim 4, wherein the cell is a Xenopus oocyte.

6. The cell of claim 4, wherein the cell is a human embryonic kidney cell.

7. The cell of claim 4, wherein said DNA expresses a neuronal calcium channel including a polypeptide subunit having the sequence presented as SEQ ID NO: 2.

8. The cell of claim 7, which further expresses neuronal calcium channel subunits α₂ and β.

9. A method of screening a compound capable of blocking calcium uptake in human neuronal cells, comprising
exposing the cell of claim 7 with the test compound,
examining the effect of said exposing on calcium uptake into the cells, and
selecting the compound if said exposing inhibits calcium uptake into the cells.

10. The method of claim 9, wherein said exposing includes contacting the cells with the cell of claim 8.

## Patentansprüche

1. Isoliertes DNA-Fragment, das eine Nukleotidsequenz umfasst, die eine alpha-1E-Untereinheit eines menschlichen neuronalen Calcium-Kanals kodiert, und die in SEQ ID NO:1 gezeigte Sequenz besitzt.

2. Alpha-1E-Polypeptid-Untereinheit eines menschlichen neuronalen Calcium-Kanals mit der in SEQ ID NO:2 gezeigten Sequenz.

3. Säuger-Expressionsvektor, enthaltend die in SEQ ID NO:1 gezeigte Nukleotidsequenz.

4. Eukaryontische Zelle, umfassend eine heterologe DNA mit der in SEQ ID NO:1 gezeigten Sequenz.

5. Zelle gemäß Anspruch 4, wobei die Zelle eine Xenopus-Oocyte ist.

6. Zelle gemäß Anspruch 4, wobei die Zelle eine menschliche embryonale Nierenzelle ist.

7. Zelle gemäß Anspruch 4, wobei die DNA einen neuronalen Calcium-Kanal exprimiert, umfassend eine Polypeptid-Untereinheit mit der in SEQ ID NO:2 gezeigten Sequenz.

8. Zelle gemäß Anspruch 7, die außerdem neuronale Calcium-Kanal-Untereinheiten α₂ und β exprimiert.

9. Verfahren zum Screening einer Verbindung, die zur Blockierung der Calciumaufnahme in menschliche neuronale Zellen fähig ist, umfassend
Exponieren der Zelle gemäß Anspruch 7 gegenüber der Testverbindung,
Untersuchung der Wirkung der Exposition auf die Calciumaufnahme in die Zellen und
Auswahl der Verbindung, falls die Exposition die Calciumaufnahme in die Zellen hemmt.

10. Verfahren gemäß Anspruch 9, wobei die Exposition ein Kontaktieren der Zellen mit der Zelle gemäß Anspruch 8 umfasst.

## Revendications

1. Fragment d'ADN isolé, comprenant une séquence de nucléotides qui code pour une sous-unité alpha-1E d'un canal calcique neuronal humain, et ayant la séquence présentée en tant que SEQ ID NO:1.

2. Sous-unité polypeptidique alpha-1E d'un canal calcique neuronal humain ayant la séquence présentée en tant que SEQ ID NO:2.

3. Vecteur d'expression mammalien contenant la séquence nucléotidique présentée en tant que SEQ ID NO:1.

4. Cellule eucaryote, qui inclut un ADN hétérologue ayant la séquence présentée en tant que SEQ ID NO:1.

5. Cellule selon la revendication 4, dans laquelle la cellule est un oocyte de Xenopus.

6. Cellule selon la revendication 4, dans laquelle la cellule est une cellule rénale embryonnaire humaine.

7. Cellule selon la revendication 4, dans laquelle ledit ADN exprime un canal calcique neuronal comprenant une sous-unité polypeptidique ayant la séquence présentée en tant que SEQ ID NO:2.

8. Cellule selon la revendication 7, qui exprime en outre des sous-unités de canal calcique neuronal α₂ et β.

9. Procédé pour la détermination d'un composé capable de bloquer la fixation du calcium dans des cellules neuronales humaines, comprenant
l'exposition de la cellule selon la revendication 7 avec le composé d'essai,
l'examen de l'effet de ladite exposition sur la fixation du calcium dans les cellules, et
la sélection du composé si ladite exposition inhibe la fixation du calcium dans les cellules.

10. Procédé selon la revendication 9, dans lequel ladite exposition comprend la mise en contact des cellules avec la cellule selon la revendication 8.
